# EUROPEAN PATENT APPLICATION

(11) **EP 2 913 769 A1**
(43) Date of publication of application: **02.09.2015**
(21) Application number: 14191015.8
(22) Date of filing: 30.10.2014
(51) Int. Cl.: G06F 19/00, A61B 8/00

(54) **Apparatus and method of processing a medical image by using a plurality of input units**

(30) Priority: 28.02.2014 KR 20140024656
(71) Applicant: Samsung Medison Co., Ltd., Gangwon-do 250-870 (KR)
(72) Inventor: Lee, Jun-kyo, Gangwon-do (KR); Kim, Sung-yoon, Gangwon-do (KR); Ahn, Mi-jeong, 250-870 Gangwon-do (KR); Jin, Gil-ju, Gangwon-do (KR)
(74) Representative: Frey, Sven Holger

(57) **Abstract**

Provided is an apparatus for processing a medical image, including: an input module including a plurality of input units; and a controller that performs a first combination function defined by a combination of a first input unit and a second input unit among the plurality of input units when user inputs through the first and second input units are detected, wherein the first combination function is related to a function defined in at least one of the first and second input units.

## Description

### RELATED APPLICATION

This application claims the benefit of Korean Patent Application No. 10-2014-0024656, filed on February 28, 2014, in the Korean Intellectual Property Office, the disclosure of which is incorporated herein in its entirety by reference.

### BACKGROUND

### 1. Field

One or more embodiments of the present invention relate to a method and apparatus for processing medical images and a computer-readable recording medium having stored thereon computer program codes that perform the method of processing medical images.

### 2. Description of the Related Art

Medical image processing systems provide various functions such as capturing, viewing, storing, and processing of medical images. As a medical image processing system provides diverse functions, the structure of an input unit for manipulating the medical imaging processing system becomes more diverse and complex. Furthermore, since a medical image processing system usually needs to input manipulation signals at high speed while capturing a medical image, it may require a large number of input units. However, the presence of a large number of input units may pose challenges to decreasing the size of the medical imaging processing system.

### SUMMARY

One or more embodiments of the present invention include a method and apparatus for processing a medical image, which are capable of performing various functions by using a small number of input units in an input module. One or more embodiments of the present invention include.

One or more embodiments of the present invention include a method and apparatus for processing a medical image, which are capable of performing various functions by using an intuitive combination of input units in an input module.

Additional aspects will be set forth in part in the description which follows and, in part, will be apparent from the description, or may be learned by practice of the presented embodiments.

According to one or more embodiments of the present invention, an apparatus for processing a medical image includes an input module including a plurality of input units; and a controller that performs a first combination function defined by a combination of a first input unit and a second input unit among the plurality of input units when user inputs through the first and second input units are detected, wherein the first combination function is related to a function defined in at least one of the first and second input units.

The first combination function may be related to a first function defined in the first input unit and a second function defined in the second input unit.

The first combination function may be related to a first function defined in the first input unit, and the second input unit may be located within a first reference distance from the first input unit.

A distance between the first and second input units may be less than a second reference distance.

The apparatus may further include a touch input detector that recognizes fingers arranged adjacent to the apparatus and detects touch inputs through the fingers, wherein the controller performs the first combination function according to a combination of the fingers through which the touch inputs are detected.

The controller may define a primary function for each of the recognized fingers, define, when a touch input through a first finger is detected, secondary functions for the remaining fingers according to the primary function of the first finger, and perform a secondary function corresponding to a second finger in response to a touch input through the second finger.

The controller may perform the first combination function when a user input through the first and second input units is detected within a reference time.

The input module may be a touch screen.

According to one or more embodiments of the present invention, a method of processing a medical image includes: detecting a user input through a first input unit and a second input unit among the plurality of input units; and performing a first combination function defined by a combination of the first and second input units, wherein the first combination function is related to a function defined in at least one of the first and second input units.

The first combination function may be related to a first function defined in the first input unit and a second function defined in the second input unit.

The first combination function may be related to a first function defined in the first input unit, and the second input unit may be located within a first reference distance from the first input unit.

A distance between the first and second input units may be less than a second reference distance.

The method may further include: recognizing fingers arranged adjacent to an apparatus for performing the method; detecting touch inputs through the fingers; and performing the first combination function according to a combination of the fingers through which the touch inputs are detected.

The method may further include: defining a primary function for each of the recognized fingers; defining, when a touch input through a first finger is detected, secondary functions for the remaining fingers according to the primary function of the first finger; and performing a secondary function corresponding to a second finger in response to a touch input through the second finger.

In the performing of the first combination function, the first combination function may be performed when a user input through the first and second input units is detected within a reference time.

The plurality of input units may be arranged on a touch screen.

According to one or more embodiments of the present invention, a non-transitory computer-readable recording medium has recorded thereon computer program codes, which, when read and executed by a processor, performs a method of processing a medical image by using a plurality of input units. The method includes: detecting a user input through a first input unit and a second input unit among the plurality of input units; and performing a first combination function defined by a combination of the first and second input units, wherein the first combination function is related to a function defined in at least one of the first and second input units.

According to the embodiments of the present invention, an input module of a medical image processing apparatus may perform various functions by using only a small number of input units therein.

Furthermore, the input module may perform various functions by using an intuitive combination of input units therein.

### BRIEF DESCRIPTION OF THE DRAWINGS

These and/or other aspects will become apparent and more readily appreciated from the following description of the embodiments, taken in conjunction with the accompanying drawings in which:
FIG. 1 is a diagram of an apparatus for processing a medical image according to an exemplary embodiment of the present invention;
FIG. 2 illustrates an input module and input units in the input module according to an exemplary embodiment of the present invention;
FIG. 3 illustrates an example of a neighboring key according to an exemplary embodiment of the present invention;
FIG. 4 is a flowchart of a method of processing a medical image according to an exemplary embodiment of the present invention;
FIG. 5 is a diagram of an apparatus for processing a medical image according to another exemplary embodiment of the present invention;
FIG. 6 illustrates a touch screen interface according to an exemplary embodiment of the present invention;
FIG. 7 illustrates a configuration of an apparatus for processing a medical image according to another exemplary embodiment of the present invention;
FIG. 8 illustrates a process of performing an input using a user's fingers according to an exemplary embodiment of the present invention;
FIG. 9 is a flowchart of a method of processing a medical image according to another exemplary embodiment of the present invention;
FIG. 10 illustrates a process of performing an input using a user's fingers according to another exemplary embodiment of the present invention;
FIG. 11 is a flowchart of a method of processing a medical image according to another exemplary embodiment of the present invention; and
FIG. 12 is a block diagram of a configuration of an ultrasound diagnostic device related to an embodiment of the present invention.

### DETAILED DESCRIPTION

The terms used in this specification are those general terms currently widely used in the art in consideration of functions in regard to the present invention, but the terms may vary according to the intention of those of ordinary skill in the art, precedents, or new technology in the art. Also, specified terms may be selected by the applicant, and in this case, the detailed meaning thereof will be described in the detailed description of the invention. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element but may further include another element. In addition, terms such as "... unit", "... module", or the like refer to units that perform at least one function or operation, and the units may be implemented as hardware or software or as a combination of hardware and software. Expressions such as "at least one of," when preceding a list of elements, modify the entire list of elements and do not modify the individual elements of the list.

Throughout the present specification, an "object" may include a person or an animal, or a part of a person or an animal. For example, the object may include the liver, the heart, the womb, the brain, a breast, the abdomen, or a blood vessel. Furthermore, the "object" may include a phantom. The phantom means a material having a volume that is approximately the density and effective atomic number of a living organism.

Furthermore, in the present specification, a "user" refers to a medical professional, such as a doctor, a nurse, a medical laboratory technologist, a medical imaging expert, and a technician who repairs a medical apparatus, but the user is not limited thereto.

Embodiments of the invention now will be described more fully hereinafter with reference to the accompanying drawings, in which illustrative embodiments of the invention are shown.

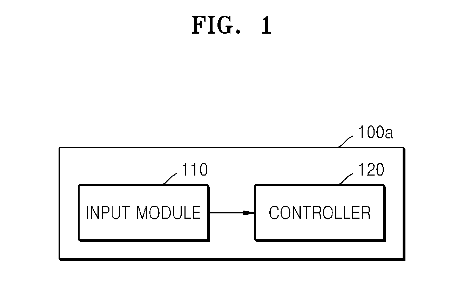

FIG. 1 is a diagram of an apparatus 100a for processing a medical image (hereinafter called a 'medical image processing apparatus') according to an exemplary embodiment of the present invention. The medical image processing apparatus 100a includes an input module 110 and a controller 120.

The input module 110 includes a plurality of input units. A user may input various control signals via the input module 110. Each of the plurality of input units may have a function assigned thereto. For example, the plurality of input units may correspond respectively to functions, i.e., capturing a color image, capturing a power Doppler (PD) image, capturing a Doppler image, measuring a pulse wave (PW), selecting a patient, executing a setting function, ending examination, two-dimensional (2D) mode, three-dimensional (3D) mode, four-dimensional (4D) mode, selecting a user, selecting a focus, eraser, selecting a probe, printing a picture, moving a pointer, measuring a parameter, body marker on/off, save function, etc.

The controller 120 controls overall operations of the medical image processing apparatus 100a. When a user input through first and second input units among the plurality of input units in the input module 110 is detected, the controller 120 may perform a first combination function defined by a combination of the first and second input units. The first combination function may be related to a function defined in at least one of the first and second input units.

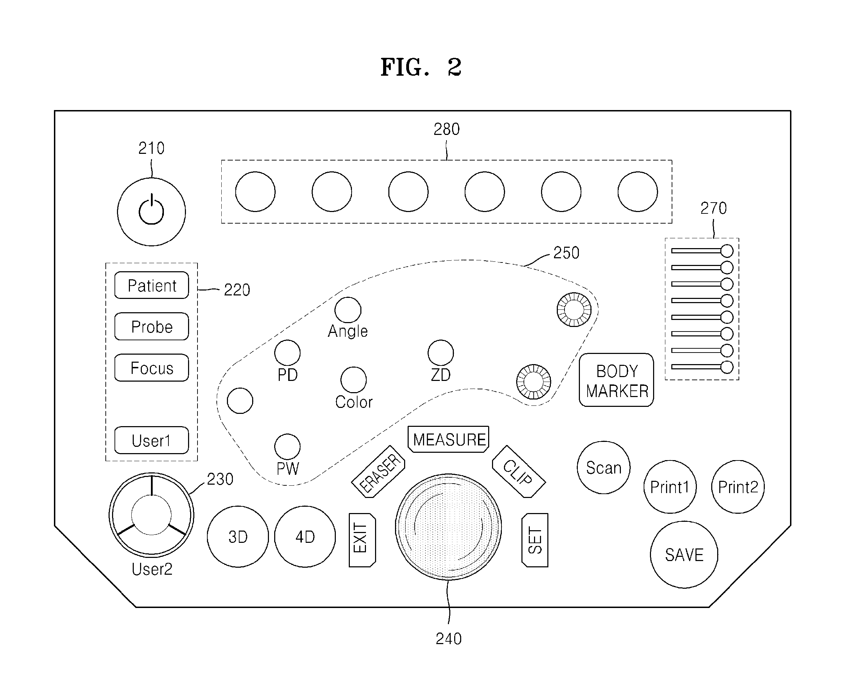

FIG. 2 illustrates the input module 110 shown in FIG. 1 and input units in the input module 110 according to an exemplary embodiment of the present invention.

Referring to FIG. 2, the input module 110 includes a plurality of input units. The plurality of input units may include a power button 210, key buttons 220, a knob button 230, a trackball 240, a dial 250, a tuner 270, and a slide bar 280.

The power button 210 is used to turn on or off the power to the medical image processing apparatus 100a.

The key button 220 is pressed to generate a manipulation signal.

The knob button 230 is pressed in a direction, i.e., up, down, left, or right to generate a manipulation signal corresponding to the direction.

The trackball 240 generates a manipulation signal by rotating a ball therein.

The dial 250 is rotated to generate a manipulation signal.

The tuner 270 generates a manipulation signal by moving protrusions thereof along a track.

The slide bar 280 is a knob with no function designated therein. A function of the slide bar 280 may be designated automatically according to an operation mode of the medical image processing apparatus 10a or manually based on a user's setting.

Each of the input units has a predetermined function assigned thereto. For example, functions for selecting a patient and selecting a probe may be assigned to a Patient key and a Probe key, respectively. Functions for capturing or reproducing 3D images and for moving a pointer may also be assigned to a 3D key and the trackball 240, respectively.

According to embodiments of the present invention, when a user input through two or more input units is detected, the controller 120 may perform a first combination function related to at least one of the two or more input units. The first combination function may be related to all of the two or more input units. Tables 1 through 5 illustrate examples of the first combination function related to all of the two or more input units.

**Table 1: Functions related to eraser**

| First input unit | Second input unit | First combination function |
|---|---|---|
| Eraser | Color | Turn on or off a color image in the entire image |
| Eraser | PD | Turn on or off a PD image in the entire image |
| Eraser | Doppler | Turn on or off a Doppler image in the entire image |
| Eraser | Elasto | Turn on or off an Elasto image (tissue elasticity image) in the entire image |
| Eraser | 2D | Turn on or off a brightness (B) mode image in the entire image |
| Eraser | 3D | Smooth cut (remove an obstacle on an object from a 3D image gradually) |
| Eraser | Measure | Erase a measured parameter |
| Eraser | Annotation | Erase annotation |
| Eraser | Patient | Erase patient information |
| Eraser | BodyMarker | Erase body marker |

**Table 2: Functions related to Patient**

| First input unit | Second input unit | First combination function |
|---|---|---|
| Patient | Set | New Exam |
| Patient | Exit | End Exam |

**Table 3: Functions related to Probe**

| First input unit | Second input unit | First combination function |
|---|---|---|
| Probe | 1D | Sequentially select a 1D probe port |
| Probe | 2D | Sequentially select a 2D probe port |
| Probe | 3D | Sequentially select a 3D probe port |

**Table 4: Functions related to Focus**

| First input unit | Second input unit | First combination function |
|---|---|---|
| Focus | 2D | When screens in a plurality of modes overlap one another, the remaining images other than a 2D image are made blurry. |
| Focus | Color | When screens in a plurality of modes overlap one another, the remaining images other than a color image are made blurry. |
| Focus | PD | When screens in a plurality of modes overlap one another, the remaining images other than a PD image are made blurry. |
| Focus | PW | When screens in a plurality of modes overlap one another, the remaining images other than a PW image are made blurry. |

**Table 5: Functions related to a trackball**

| First input unit | Second input unit | First combination function |
|---|---|---|
| Trackball | 3D | 3D cine mode |
| Trackball | 4D | 4D cine mode |

According to another embodiment, the first combination function may be formed by combining a first input unit with a second input unit that is adjacent to the first input unit, and may be related to a function defined in the first input unit. Table 6 illustrates examples of a first combination function defined by the first input unit and its neighboring input unit as the second input unit.

**Table 6**

| First input unit | Second input unit | First combination function |
|---|---|---|
| Save | Neighboring key 1 | Save an image |
| Save | Neighboring key 2 | Save a clip |
| Probe | Neighboring key 1 | Select a probe of a first port |
| Probe | Neighboring key 2 | Select a probe of a second port |

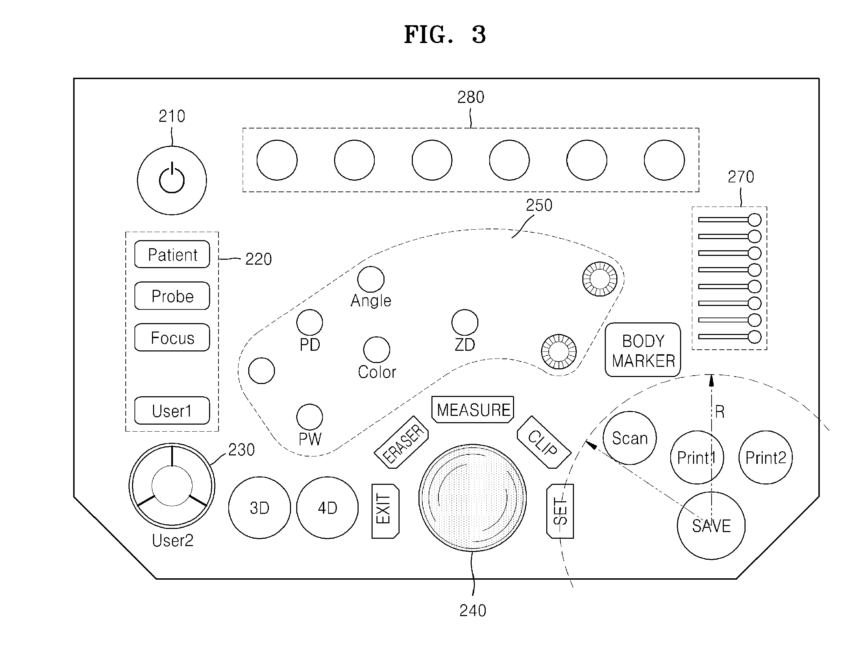

FIG. 3 illustrates an example of a neighboring key according to an exemplary embodiment of the present invention.

The neighboring key according to the present embodiment may be selected from among keys arranged within a first reference distance R from a first input unit. For example, as shown in FIG. 3, if the first input unit is a SAVE key, the neighboring key may be selected from among SCAN, PRINT1, PRINT2, and SET keys that are located within the first reference distance R from the SAVE key.

Furthermore, in one embodiment, the first and second input units may be arranged within a second reference distance from each other. For example, the second reference distance may be determined based on a general size of a user's hand.

The first combination function may be defined regardless of the order in which user inputs are fed via the first and second input units. In another embodiment, the first combination function may be defined in consideration of this order.

According to one embodiment, the first combination function may be executed only when user inputs are fed through the first and second input units within a reference time. The reference time may be set by a user.

In one embodiment, the first combination function may be defined for user inputs using three or more input units.

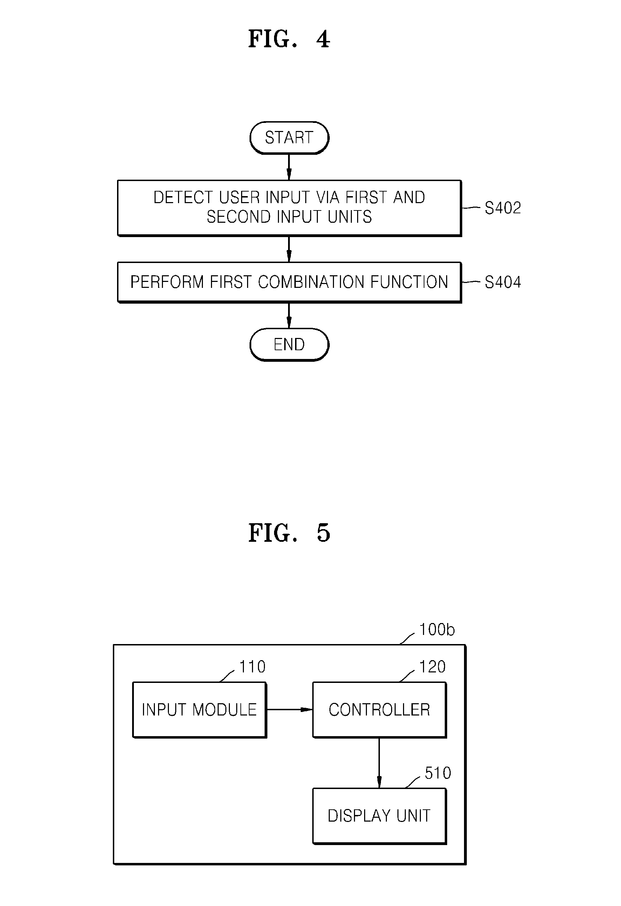

FIG. 4 is a flowchart of a method of processing a medical image according to an exemplary embodiment of the present invention

The method according to the present embodiment is performed by using a medical image processing apparatus including a plurality of input units. According to the method, when user inputs via first and second input units among the plurality of input units are detected (S402), a first combination function is performed (S404).

At least one of the first and second input units may have a function assigned thereto. The first combination function is related to the function assigned to the at least one of the first and second input units.

In one embodiment, the first combination function may be related to functions assigned to both the first and second input units.

In another embodiment, the first combination function is related to the first input unit, and the second input unit may be located at a distance less than a first reference distance from the first input unit.

FIG. 5 is a diagram of a medical image processing apparatus 100b according to another exemplary embodiment of the present invention. Referring to FIG. 5, the medical image processing apparatus 100b includes an input module 110, a controller 120, and a display unit 510.

The input module 110 includes a plurality of input units. The user may input various control signals via the input module 110. Each of the plurality of input units may have a function assigned thereto.

The controller 120 controls overall operations of the medical image processing apparatus 100b. Upon detecting user inputs via first and second input units among the plurality of input units in the input module 110, the controller 120 may perform a first combination function that is defined by a combination of the first and second input units. The first combination function may be related to a function assigned to at least one of the first and second input units.

The display unit 510 may display captured medical images and various information that is processed by the medical image processing apparatus 100b. The display unit 510 may be realized as a touch screen.

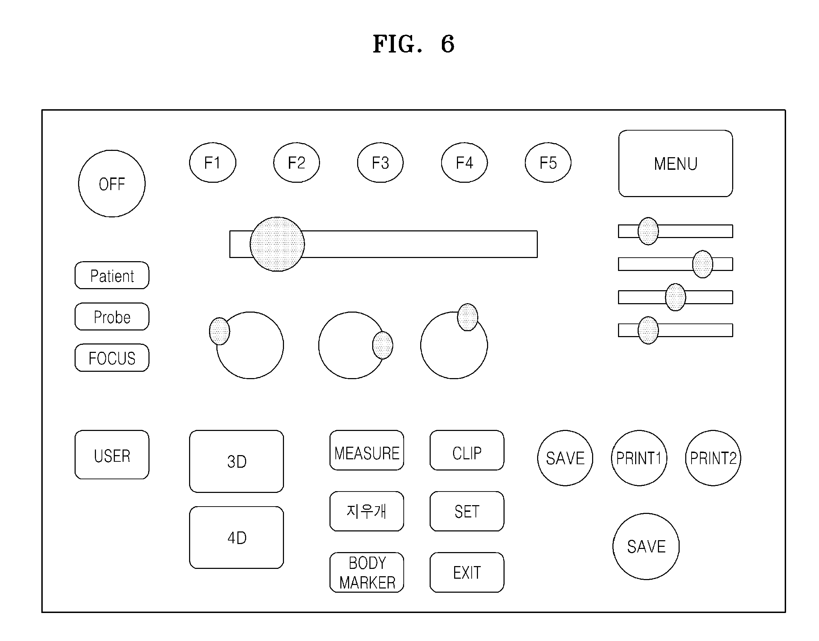

FIG. 6 illustrates a touch screen interface according to an exemplary embodiment of the present invention.

According to the present embodiment, the input module 110 may be provided as a touch screen interface that is displayed on the display unit 510. The touch screen interface of FIG. 6 may include a plurality of input units having functions assigned thereto.

A user may input a manipulation signal through two or more input units on a touch screen interface. The input module 110 may detect two or more touch inputs through a touch screen. In response to an input through the first and second input units on the touch screen interface, the controller 120 may perform a first combination function corresponding to a combination of the first input unit and the second input unit.

The touch screen interface may be implemented by using a touch screen that enables multiple touches.

According to another embodiment, the touch screen interface may be controlled by using a touch pad. In this case, the touch pad may enable multiple touches.

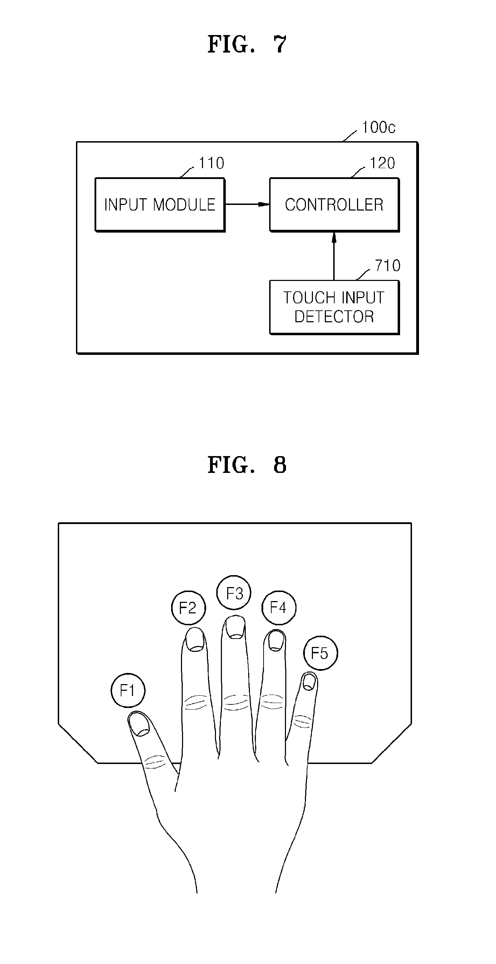

FIG. 7 illustrates a configuration of a medical image processing apparatus 100c according to another exemplary embodiment of the present invention. The medical image processing apparatus 100c includes an input module 110, a controller 120, and a touch input detector 710.

The input module 110 includes a plurality of input units. The user may input various control signals via the input module 110. Each of the plurality of input units may have a function assigned thereto.

The touch input detector 710 recognizes fingers arranged adjacent to the medical image processing apparatus 100c and detects touch inputs through the fingers.

In one embodiment, the touch input detector 710 may recognize fingers arranged adjacent to the plurality of input units in the input module 110. In another embodiment, the touch input detector may recognize fingers that are adjacent to a housing of the medical image processing apparatus 100c. In another embodiment, the touch input detector 710 may recognize fingers arranged on a display unit (not shown) of the medical image processing apparatus 100c. According to one embodiment, the touch input detector 710 may be configured to recognize the fingers even if the fingers do not touch the medical image processing apparatus 100c. Alternatively, the touch input detector 710 may be configured to recognize the fingers when the fingers simultaneously touch the medical image processing apparatus 100c.

When fingers are recognized when the fingers does not contact the medical image processing apparatus 100c, a motion recognition controller may be used. For example, the motion recognition controller may be implemented by using Leap Motion, a red, green, and blue (RGB) camera, an infrared camera, a thermal detecting camera, etc.

The touch input detector 710 may recognize the shape and type of each finger (e.g., thumb, index finger, middle finger, ring finger, or little finger). Furthermore, the touch input detector 710 may track movement of each finger to detect which finger is used to cause a touch input.

The touch input detector 710 may recognize fingers arranged adjacent to the medical image processing apparatus 100c and then detect touch inputs through the recognized fingers. For example, the touch input detector 710 may detect a touch input by using a capacitive or resistive method.

The controller 120 controls overall operations of the medical image processing apparatus 100c.

When a user input through first and second input units among a plurality of input units in the input module 110 is detected, the controller 120 performs a first combination function defined by a combination of the first and second input units. The first combination function may be related to a function defined in at least one of the first and second input units.

Furthermore, the controller 120 performs a first combination function by using a combination of fingers through which a touch input is detected. For example, if a touch input using a thumb and an index finger is detected, a captured image may be stored. If a touch input using a thumb and a middle finger is detected, a medical image may be printed.

FIG. 8 illustrates a process of performing an input using a user's fingers according to an exemplary embodiment of the present invention.

According to the present embodiment, when it is recognized that fingers are arranged adjacent to the medical image processing apparatus (100c in FIG. 7), the controller 120 may assign primary functions F1 through F5 to the fingers, respectively. The primary functions F1 through F5 may be determined automatically or by a user's setting. When the touch input detector 710 is realized as a touch screen, icons representing the primary functions F1 through F5 may be displayed on the touch screen.

When a touch input through a finger is detected, the controller 120 may perform a primary function assigned to the finger. In one embodiment, when a touch input using a thumb and an index finger is detected, a first combination function related to primary functions F1 and F2 may be performed.

According to another embodiment, an input may be performed using fingers of both hands. In this case, the touch input detector 710 may recognize fingers of the left and right hands and assign primary functions to the fingers, respectively.

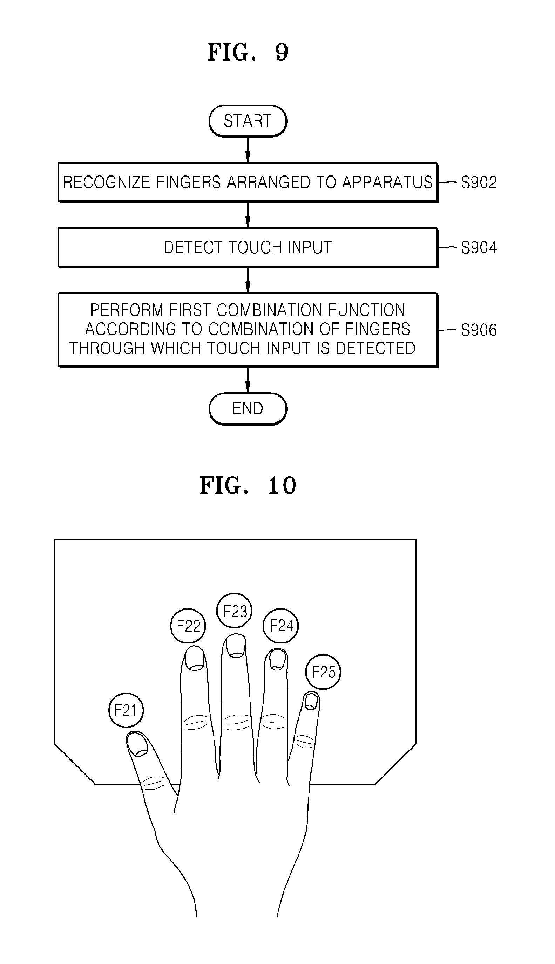

FIG. 9 is a flowchart of method of processing a medical image according to another exemplary embodiment of the present invention.

Referring to FIG. 9, fingers arranged adjacent to the medical image processing apparatus 100c are recognized (S902). Then, touch inputs through the recognized fingers are detected (S904). When the touch inputs are detected, primary functions are assigned to the fingers, respectively, a first combination function is performed by using a combination of the fingers through which the touch inputs are detected (S906).

FIG. 10 illustrates a process of performing an input using a user's fingers according to another exemplary embodiment of the present invention.

According to the present embodiment, when a touch input through a first finger is detected, secondary functions corresponding to the remaining fingers are defined according to a function assigned to the first finger, and one of the secondary functions corresponding to a second finger is performed in response to a touch input using the second finger. For example, as shown in FIG. 10, if a touch input through an index finger is detected, the secondary functions F21, F23, F24, and F25 other than the primary functions F1, F3, F4, F5 assigned to the index finger may be assigned to the remaining fingers, i.e., a thumb, a middle finger, a ring finger, and a little finger, respectively. In the present embodiment, a user may perform various functions by using the user's fingers.

In one embodiment, the touch input through the second finger needs to be detected while the touch input through the first finger is being detected. In another embodiment, the touch input through the second finger has to be detected in less than a predetermined time after the touch input via the first finger is detected.

In another embodiment, an input may be performed using fingers of both hands.

In another embodiment, when a touch input through the first finger is detected, secondary functions may be defined for all fingers including the first finger. In this case, the secondary functions may be performed only when a second input through a finger is detected within a reference time after detection of the touch input through the first finger.

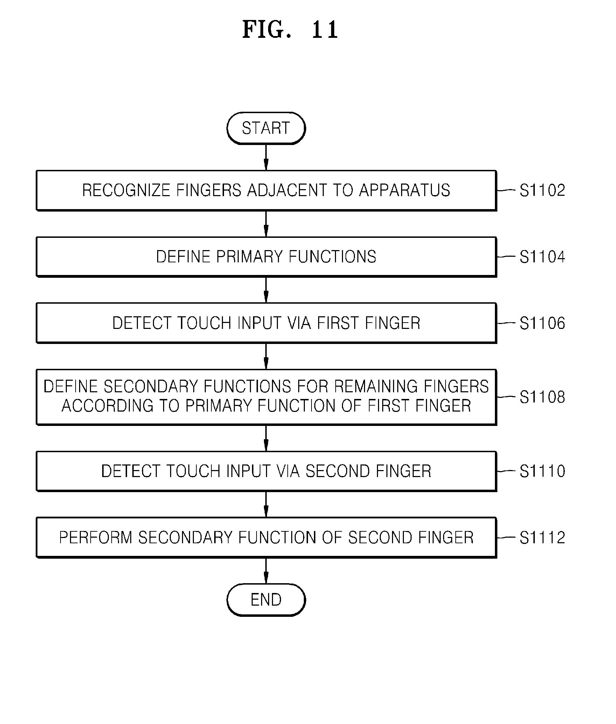

FIG. 11 is a flowchart of a method of processing a medical image according to another exemplary embodiment of the present invention.

Referring to FIG. 11, first, fingers arranged adjacent to a medical image processing apparatus are recognized (S1102). Then, primary functions are defined for the recognized fingers, respectively (S1104).

If a touch input through a first finger is detected (S1106), secondary functions are defined for the remaining fingers according to a primary function assigned to the first ringer (S1108). Then, when a touch input through a second finger is detected (S1110), a secondary function assigned to the second finger is performed (S1112).

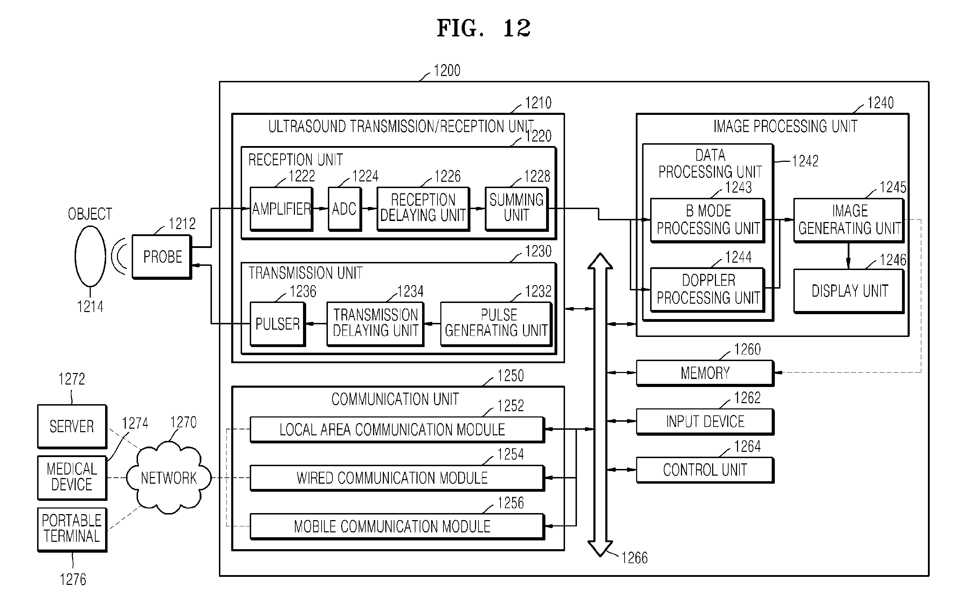

FIG. 12 is a block diagram of a configuration of an ultrasound diagnostic device 1200 related to an embodiment of the present invention. The medical image processing apparatuses 100a, 100b, and 100c according to the embodiments of the present invention may be realized as the ultrasound diagnostic device 1200.

The ultrasound diagnostic device 1200 according to the present embodiment may include a probe 1212, an ultrasound transmission/reception unit 1210, an image processing unit 1240, a communication unit 1250, a memory 1260, an input device 1262, and a controller 1264, and the components may be connected to one another via buses 1266.

The ultrasound diagnostic device 1200 may be embodied not only as a cart type device but also as a portable type. Examples of portable ultrasound diagnostic devices may include a PACS viewer, a smartphone, a laptop computer, a personal digital assistant (PDA), and a tablet PC. However, the present invention is not limited thereto.

The probe 1212 transmits ultrasound signals to an object 1214 based on a driving signal applied by the ultrasound transmission/reception unit 1210 and receives echo signals reflected from the object 1214. The probe 1212 includes a plurality of transducers, and the plurality of transducers oscillate based on electric signals transmitted thereto and generate acoustic energy, that is, ultrasound waves. Furthermore, the probe 1212 may be connected to a main body of the ultrasound diagnostic device 1200 by wires or wirelessly. According to embodiments of the present invention, the ultrasound diagnostic device 1200 may include a plurality of probes 1112.

A transmission unit 1230 supplies a driving signal to the probe 1212 and includes a pulse generating unit 1232, a transmission delaying unit 1234, and a pulser 1236. The pulse generating unit 1232 generates pulses for forming transmission ultrasound waves based on a predetermined pulse repetition frequency (PRF), and the transmission delaying unit 1234 applies a delay time for determining transmission directionality to the pulses. Pulses, to which a delay time is applied, correspond to a plurality of piezoelectric vibrators included in the probe 1212, respectively. The pulser 1236 applies a driving signal (or a driving pulse) to the probe 1212 at a timing corresponding to each pulse to which a delay time is applied.

A reception unit 1220 generates ultrasound data by processing echo signals received from the probe 1212. The reception unit 1120 may include an amplifier 1222, an analog-to-digital converter (ADC) 1224, a reception delaying unit 1226, and a summing unit 1228. The amplifier 1222 amplifies echo signals in each channel, and the ADC 1224 performs analog-to-digital conversion on the amplified echo signals. The reception delaying unit 1226 applies delay times for determining reception directionality to the echo signals subjected to the analog-to-digital conversion, and the summing unit 1228 generates ultrasound data by summing the echo signals processed by the reception delaying unit 1226. According to embodiments of the present invention, the reception unit 1220 may not include the amplifier 1222. In other words, if the sensitivity of the probe 1212 or the capability of the ADC 1224 to process bits is enhanced, the amplifier 1222 may be omitted.

The image processing unit 1240 generates an ultrasound image by scan-converting ultrasound data generated by the ultrasound transmission/reception unit 1210 and displays the ultrasound image. In addition, an ultrasound image may include not only a gray-scale ultrasound image obtained by scanning an object in an amplitude (A) mode, a B mode, and a motion (M) mode, but also a Doppler image representing a moving object by using a Doppler effect. The Doppler image may include a blood flow Doppler image (also called a color Doppler image) showing a flow of blood, a tissue Doppler image showing movement of tissue, and a spectral Doppler image showing a moving speed of an object as a waveform.

A B mode processing unit 1243 extracts B mode components from ultrasound data and processes the B mode components. An image generating unit 1245 may generate an ultrasound image indicating signal intensities as brightness based on the extracted B mode components.

Similarly, a Doppler processing unit 1244 may extract Doppler components from ultrasound data, and the image generating unit 1245 may generate a Doppler image indicating movement of an object as colors or waveforms based on the extracted Doppler components.

The image generating unit 1245 according to an embodiment of the present invention may generate a 3D ultrasound image via volume-rendering of volume data and an elasticity image which visualizes the degree of deformation of the object 1214 due to pressure. Furthermore, the image generating unit 1245 may display various additional information in an ultrasound image by using text and graphics. In addition, the generated ultrasound image may be stored in the memory 1260.

A display unit 1246 displays and outputs the generated ultrasound image. The display unit 1246 may display and output not only an ultrasound image but also various information processed by the ultrasound diagnostic device 1200 on a screen via a graphical user interface (GUI). In addition, the ultrasound diagnostic device 1200 may include two or more display units 1246 according to embodiments of the present invention.

The communication unit 1250 is connected to a network 1270 by wires or wirelessly and communicates with an external device or a server. The communication unit 1250 may exchange data with a hospital server or another medical device in a hospital that is connected via a picture archiving and communications system (PACS). Furthermore, the communication unit 1250 may perform data communication according to the digital imaging and communications in medicine (DICOM) standard.

The communication unit 1250 may transmit or receive data related to diagnosis of the object 1214, e.g., an ultrasound image, ultrasound data, and Doppler data of the object 1214, via the network 1270. The communication unit 1250 may also transmit or receive medical images obtained by other medical devices, such as a computed tomography (CT) image, a magnetic resonance (MR) image, and an X-ray image. Furthermore, the communication unit 1250 may receive information related to a diagnosis history or a treatment schedule of a patient from a server and utilizes the information for diagnosing the patient. Furthermore, the communication unit 1250 may perform data communication with a server or a medical device in a hospital as well as a portable terminal of a doctor or a patient.

The communication unit 1250 is connected to the network 1270 in a wired or wireless manner and may exchange data with a server 1272, a medical device 12744, or a portable terminal 1276. The communication unit 1250 may include at least one component that enables communication with external devices, e.g., a local area communication module 1252, a wired communication module 1254, and a mobile communication module 1256.

The local area communication module 1252 is a module for performing local area communication with a device within a predetermined distance. Examples of local area communication technology include a wireless Local Area Network (LAN), Wi-Fi, Bluetooth, ZigBee, Wi-Fi Direct (WFD), Ultra Wideband (UWB), Infrared Data Association (IrDA), Bluetooth Low Energy (BLE), and Near Field Communication (NFC), but are not limited thereto.

The wired communication module 1254 is a module for performing communication by using an electric signal or an optical signal. Examples of wired communication technology include wired communication technologies using a pair cable, a coaxial cable, an optical fiber cable, and an Ethernet cable.

The mobile communication module 1256 transmits or receives wireless signals to or from at least one of a base station, an external terminal, and a server on a mobile communication network. Here, the wireless signals may include voice call signals, video call signals, or various types of data for transmission and reception of text/multimedia messages.

The memory 1260 stores various data processed by the ultrasound diagnostic device 1200. For example, the memory 1260 may store not only medical data related to the diagnosis of the object 1214, such as ultrasound data and ultrasound image that are input or output, but also algorithms or programs that are executed in the ultrasound diagnostic device 1200.

The memory 1260 may be embodied as any of various storage media such as a flash memory, a hard disk drive, and an Electrically Erasable Programmable Read-Only Memory (EEPROM). Furthermore, the ultrasound diagnostic device 1200 may utilize a web storage or a cloud server that functions as the memory 1260 online.

The input device 1262 is a means via which a user inputs data for controlling the ultrasound diagnostic device 1200. The input device 1262 may include hardware components, such as a keypad, a mouse, a touch panel, a touch screen, and a jog switch. However, the present invention is not limited thereto, and the input device 1262 may further include various other input elements such as an electrocardiogram measuring module, a respiration measuring module, a voice recognition sensor, a gesture recognition sensor, a fingerprint recognition sensor, an iris recognition sensor, a depth sensor, a distance sensor, etc.

The controller 1264 may control overall operations of the ultrasound diagnostic device 1200. In other words, the controller 1264 may control operations among the probe 1212, the ultrasound transmission/reception unit 1210, the image processing unit 1240, the communication unit 1250, the memory 1260, and the input device 1262.

All or some of the probe 1212, the ultrasound transmission/reception unit 1210, the image processing unit 1240, the communication unit 1250, the memory 1260, the input device 1262, and the controller 1264 may be operated by software modules. However, the present invention is not limited thereto, and some of the above components may be operated by hardware modules. Furthermore, at least one of the ultrasound transmission/reception unit 1210, the image processing unit 1240, and the communication unit 1250 may be included in the controller 1264, but are not limited thereto.

The input module 110 according to an embodiment of the present invention may correspond to the input device 1262 shown in FIG. 12.

The controller 120 according to an embodiment of the present invention may correspond to the controller 1264 shown in FIG. 12.

The display unit 510 shown in FIG. 5 may correspond to the display unit 1246 shown in FIG. 12.

The touch input detector 710 shown in FIG. 7 may correspond to the input device 1262.

According to another embodiment of the present invention, the medical image processing apparatuses 100a, 100b, and 100c may be realized as CT or magnetic resonance imaging (MRI) diagnostic equipment.

A method of processing medical images according to an embodiment of the present invention may be implemented as a software module or algorithm. Methods implemented as software modules or algorithms may be stored on a computer-readable storage medium as computer-readable codes or program instructions that can be executed on a processor. Examples of computer-readable storage media include magnetic storage media (e.g., ROM, RAM, floppy disks, hard disks, etc.) and optical recording media (e.g., CD-ROMs, DVDs, etc.). The computer-readable storage media can also be distributed over network-coupled computer systems so that computer-readable codes are stored and executed in a distributed fashion. The computer-readable codes may be read by a computer, stored in a memory, and executed on a processor. When the storage media are connected to the medical image processing apparatus 100a, 100b, or 100c, the medical image processing apparatus 100a, 100b, or 100c may be configured to perform methods of processing medical images according to embodiments of the present invention.

While one or more embodiments of the present invention have been described with reference to the figures, it will be understood by those of ordinary skill in the art that various changes in form and details may be made therein without departing from the spirit and scope of the present invention as defined by the following claims. Thus, it should be understood that the exemplary embodiments described therein should be considered in a descriptive sense only and not for purposes of limitation.

## Claims

1. An apparatus for processing a medical image, the apparatus comprising:
an input module including a plurality of input units; and
a controller that performs a first combination function defined by a combination of a first input unit and a second input unit among the plurality of input units when user inputs through the first and second input units are detected,
wherein the first combination function is related to a function defined in at least one of the first and second input units.

2. The apparatus of claim 1, wherein the first combination function is related to a first function defined in the first input unit and a second function defined in the second input unit.

3. The apparatus of claim 1, wherein the first combination function is related to a first function defined in the first input unit, and the second input unit is located within a first reference distance from the first input unit.

4. The apparatus of claim 1, wherein a distance between the first and second input units is less than a second reference distance.

5. The apparatus of claim 1, further comprising a touch input detector that recognizes fingers arranged adjacent to the apparatus and detects touch inputs through the fingers,
wherein the controller performs the first combination function according to a combination of the fingers through which the touch inputs are detected.

6. The apparatus of claim 5, wherein the controller defines a primary function for each of the recognized fingers, defines, when a touch input through a first finger is detected, secondary functions for the remaining fingers according to the primary function of the first finger, and performs a secondary function corresponding to a second finger in response to a touch input through the second finger.

7. The apparatus of claim 1, wherein the controller performs the first combination function when a user input through the first and second input units is detected within a reference time.

8. The apparatus of claim 1, wherein the input module is a touch screen.

9. A method of processing a medical image by using a plurality of input units, the method comprising:
detecting a user input through a first input unit and a second input unit among the plurality of input units; and
performing a first combination function defined by a combination of the first and second input units,
wherein the first combination function is related to a function defined in at least one of the first and second input units.

10. The method of claim 9, wherein the first combination function is related to a first function defined in the first input unit and a second function defined in the second input unit.

11. The method of claim 9, wherein the first combination function is related to a first function defined in the first input unit, and the second input unit is located within a first reference distance from the first input unit.

12. The method of claim 9, wherein a distance between the first and second input units is less than a second reference distance.

13. The method of claim 9, further comprising:
recognizing fingers arranged adjacent to an apparatus for performing the method;
detecting touch inputs through the fingers; and
performing the first combination function according to a combination of the fingers through which the touch inputs are detected.

14. The method of claim 13, further comprising:
defining a primary function for each of the recognized fingers;
defining, when a touch input through a first finger is detected, secondary functions for the remaining fingers according to the primary function of the first finger; and
performing a secondary function corresponding to a second finger in response to a touch input through the second finger.

15. The method of claim 9, wherein in the performing of the first combination function, the first combination function is performed when a user input through the first and second input units is detected within a reference time.
